# EUROPEAN PATENT APPLICATION

(11) **EP 1 880 759 A2**
(43) Date of publication of application: **23.01.2008**
(21) Application number: 07120203.0
(22) Date of filing: 12.08.2005
(51) Int. Cl.: B01J 19/00, C12Q 1/68

(54) **Post-Synthesis Processing System for Supported Oligonucleotides, and Method**

(30) Priority: 22.09.2004 US 946718
(62) Divisional of application: 05789979.1
(71) Applicant: Applera Corporation, Foster City, CA 94404 (US)
(72) Inventor: The designation of the inventor has not yet been filed
(74) Representative: Stephen, Robert John

(57) **Abstract**

A system is provided that can include: a plurality of retainment regions, where each retainment region is adapted to retain a respective type of an oligonucleotide supported on a respective support; a mixture retainment region; a handling device; a control unit adapted to control the handling device; and a separating unit adapted to simultaneously separate different supported oligonucleotides from their respective supports. A method is provided that can include: pooling together a plurality of supported oligonucleotides to form a mixture; and simultaneously separating the oligonucleotides of the supported oligonucleotides in the mixture from their supports. A method for facilitating genetic analysis is also provided and can include: receiving from a user a request for one or more genetic analysis assays; formulating each assay; and providing to the user the one or more assay; wherein the formulating can include pooling together into a mixture retainment region a plurality of different supported oligonucleotides and simultaneously separating the different oligonucleotides from their respective supports.

## Description

### CROSS-REFERENCE TO OTHER APPLICATIONS

The present application claims a priority from U.S. Patent Application No. 10/946,718, filed September 22, 2004, which is incorporated herein in its entirety by reference. The teachings of International Publications Nos.: WO 00/49382, international filing date February 15, 2000; WO 00/48735, international filing date February 15, 2000; and WO 03/022437 A1, international filing date September 9, 2002, are all incorporated herein in their entireties by reference.

### FIELD

The present teachings relate to a post-synthesis processing system of a supported amino acid molecule, for example, a peptide, a nucleotide, a polynucleotide, a 10-mer nucleotide, a 20-mer nucleotide, or an oligonucleotide, that can be bound to a support.

### BACKGROUND

Post-synthesis processing costs of a supported oligonucleotide can be significant in making a poly-oligonucleotide assay. To be useful, a synthesized oligonucleotide might require post-synthesis processing, for example, cleaving from a support or an anchor, for example, using a wash material such as an ammonia bath. The processing might also necessarily require purifying or desalting the cleaved or uncleaved oligonucleotide, and analyzing for quality control to ensure proper length and purity of the oligonucleotide. Systems and methods capable of reducing the post-synthesis processing costs of oligonucleotides are desirable.

### SUMMARY

According to various embodiments, a system is provided that can comprise a plurality of retainment regions, a mixture retainment region, a handling device, a control unit, and a separating unit. Each of the retainment regions can be adapted to retain a respective type of chemical supported by or otherwise attached to a support. The control unit can be programmed, programmable, and/or operable to control the handling device to pool in the mixture retainment region different support chemicals from different ones of the retainment regions, to form a pool. The separating unit can be adapted to simultaneously separate the different supported chemicals from their respective supports, for example, in the mixture retainment region. The different chemicals can be chemically bonded to their respective supports, for example, by one or more of ionic bonds, covalent bonds, hydrogen bonds, and Van der Waals forces. The separating unit can be capable of breaking such bonds to separate or detach the chemicals from their supports. According to various embodiments, methods using such a system are provided whereby different types of chemicals attached to supports can be pooled together and then simultaneously cleaved or separated from their respective supports.

According to various embodiments, a system is provided that comprises: a plurality of retainment regions, where each retainment region is adapted to retain a respective type of supported oligonucleotide, for example, anchored or supported on a respective bead or particle support; a mixture retainment region; a handling device; a control unit adapted to control the handling device; and a separating unit adapted to simultaneously separate different supported oligonucleotides from respective supports. The control unit can be programmed, programmable, and/or operable to control the handling device to pool in the mixture retainment region different supported oligonucleotides from different ones of the retainment regions, and resultantly form a pool.

According to various embodiments, a method for facilitating genetic analysis is provided comprising: receiving from a user a request for one or more genetic analysis assays; providing to the user (i) the one or more assays, with each assay being provided in a single tube format, and (ii) information related to the one or more assays, with the information being in electronic form; and formulating each assay. The formulating comprises pooling together into a mixture retainment region a plurality of different oligonucleotides each supported on a respective support, and separating the different oligonucleotides, simultaneously, from their respective supports, for example, in the mixture retainment region.

Additional features and advantages of various embodiments will be set forth in part in the description that follows, and in part will be apparent from the description, or can be learned by practice of various embodiments. Other advantages of the various embodiments will be realized and attained by means of the elements and combinations particularly pointed out in the application.

### BRIEF DESCRIPTION OF THE DRAWINGS

Various embodiments of the present teachings are exemplified in the accompanying drawings. The teachings are not limited to the embodiments depicted in the drawings, and include equivalent structures and methods as set forth in the following description and as would be known to those of ordinary skill in the art in view of the present teachings. In the drawings:

Fig. 1 is a prior art post-synthesis process flow diagram;

Fig. 2 is a partial post-synthesis process flow diagram where supports for different supported oligonucleotides can be removed before shipping an assay to a user, according to various embodiments;

Fig. 3 is a partial post-synthesis process flow diagram where at least some of the supports for different supported oligonucleotides can be shipped to a user in an assay, according to various embodiments;

Fig. 4 is a partial post-synthesis process flow diagram where at least one of the different supported oligonucleotides can be stored prior to a pooling;

Fig. 5 depicts an arrangement of different supported oligonucleotides retained in an array of retainment regions, where the different supported oligonucleotides can be identified using a machine readable identifier disposed on the array;

Fig. 6 is a schematic of a handling system including an illustration of ranges of motions and motion directions that the handling system can perform with respect to a plurality of retainment regions;

Fig. 7a is a perspective view of a handling system according to various embodiments, and positioned to retrieve a supported oligonucleotide from a retainment region;

Fig. 7b is a perspective view of a handling system according to various embodiments, and depicts the extraction of a supported oligonucleotide from a retainment region by the handling system;

Fig. 7c is a perspective view of a handling system according to various embodiments, and depicts positioning a handling system in alignment with a mixture retainment region;

Fig. 7d is a perspective view of a handling system according to various embodiments, and depicts an at-rest position for the handling system, for example, after delivery of a supported oligonucleotide to a mixture retainment region; and

Fig. 8 is a schematic diagram of a handling system, a detection unit, a control unit, and a plurality of mixture retainment regions, according to various embodiments.

It is to be understood that both the foregoing general description and the following detailed description are exemplary and explanatory only and are intended to provide a further explanation of the various embodiments of the present teachings.

### DESCRIPTION OF VARIOUS EMBODIMENTS

According to various embodiments, a system is provided that can comprise a plurality of retainment regions, a mixture retainment region, a handling device, a control unit, and a separating unit. Each of the retainment regions can be adapted to retain a respective type of chemical supported by or otherwise attached to a support. The control unit can be programmed, programmable, and/or operable to control the handling device to pool in the mixture retainment region different support chemicals from different ones of the retainment regions, to form a pool. The separating unit can be adapted to simultaneously separate the different supported chemicals from their respective supports, for example, in the mixture retainment region. The different chemicals can be chemically bonded to their respective supports, for example, by one or more of ionic bonds, covalent bonds, hydrogen bonds, and Van der Waals forces. The separating unit can be capable of breaking such bonds to separate or detach the chemicals from their supports. According to various embodiments, methods using such a system are provided whereby different types of chemicals attached to supports can be pooled together and then simultaneously cleaved or separated from their respective supports.

According to various embodiments, a system is provided that comprises: a plurality of retainment regions, where each retainment region is adapted to retain a respective type of supported oligonucleotide, for example, anchored or supported on a respective bead or particle support; a mixture retainment region; a handling device; a control unit adapted to control the handling device; and a separating unit adapted to simultaneously separate different supported oligonucleotides from respective supports. The control unit can be programmed, programmable, and/or operable to control the handling device to pool in the mixture retainment region different supported oligonucleotides from different ones of the retainment regions, thereby forming a pool.

According to various embodiments, supported oligonucleotides in the present teachings can comprise an amino acid molecule, for example, a peptide, a nucleotide, a polynucleotide, a 10-mer nucleotide, a 20-mer nucleotide, or an oligonucleotide, that can be bound to a support.

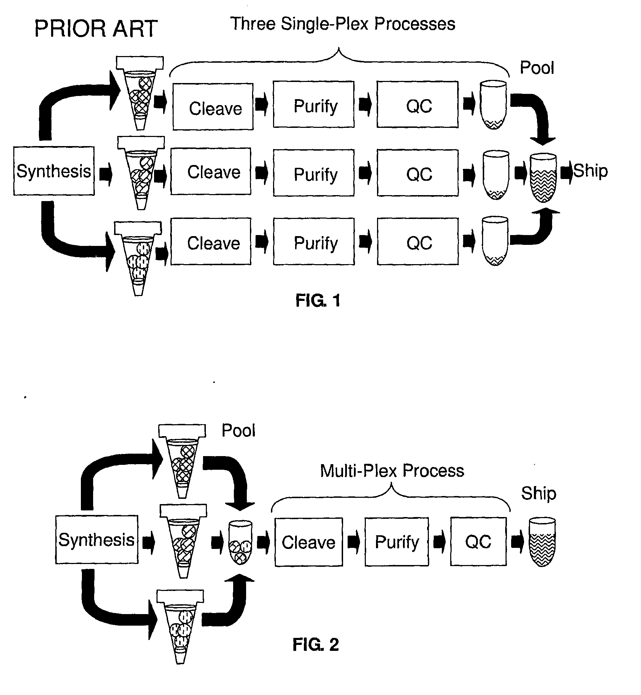

Fig. 1 is prior art post-synthesis process flow diagram of a method wherein three different oligonucleotides are synthesized. In the prior art, oligonucleotide manufacturing processes perform synthesis and post-synthesis on each oligonucleotide separately. Fig. 1 shows that three supported oligonucleotides are subjected individually to a separation or cleaving process, purification, and subsequently subjected to quality control checks, such as an inspection, to ensure proper length and purity of the respective oligonucleotide.

While this approach is necessary for oligonucleotides sold individually, it is inefficient if the oligonucleotides are to be pooled. In the prior art, different cleaved or unsupported oligonucleotides are pooled into an assay subsequent to post-processing, for example, cleaving, and are then shipped. The individual post-processing, of different oligonucleotides can incur significant manufacturing costs in a pooled product or assay utilizing such oligonucleotides.

Fig. 2 is a partial post-synthesis process flow diagram according to various embodiments of the present teachings, and wherein supports for different supported oligonucleotides can be synthesized sequentially or simultaneously. The different supported oligonucleotides can be removed and then concurrently and/or simultaneously pooled into a vessel before being shipped in an assay to a user. The post-synthesis processing reactions, for example, cleaving, purifying and quality control, can be performed after pooling of different supported oligonucleotides. The supports can be removed, if at all, during or after the post-synthesis processing of the pooled different oligonucleotide supports.

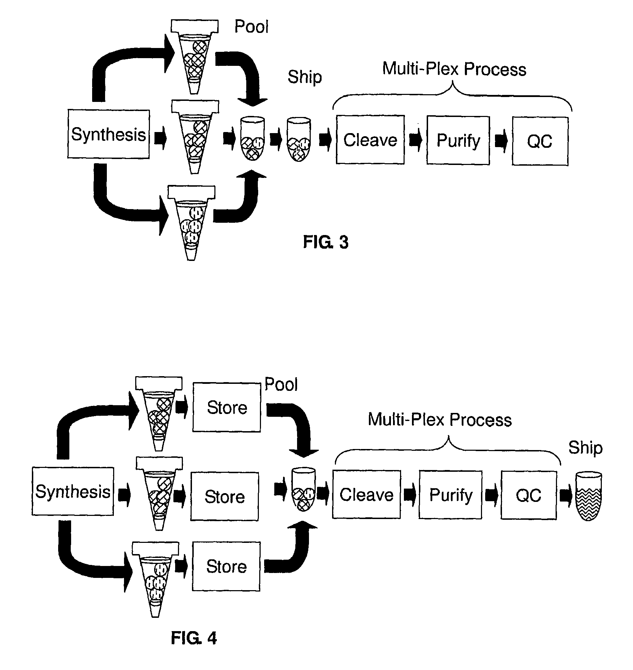

Fig. 3 is a partial process flow diagram wherein different supported oligonucleotides can be pooled and then shipped in an assay to a user. The post-synthesis processing reactions, for example, cleaving, purifying and quality control, are performed after pooling the different supported oligonucleotides. The post-synthesis processing, for example, can be performed by a user. The different oligonucleotides can be shipped in an assay to a user, along with the supports.

Fig. 4 is a partial post-synthesis process flow diagram where at least one of different supported oligonucleotides can be stored prior to pooling a desired number and/or desired types of different supported oligonucleotides. The oligonucleotides can be stored for any duration, for example, up to and including the oligonucleotide effectiveness or freshness date. Each type of supported oligonucleotide of the different supported oligonucleotides can be stored in a respective individual container or vessel. Each individual vessel can respectively be a synthesizer tube or a different vessel. The individual vessels can be stored in one or more array. The individual vessels can each be a single vessel, where each single vessel is formed in or disposed in an array of vessels. The vessels and/or the arrays can be addressable, for example, such that the control unit can be programmed to control the handling device to select and mix together appropriate supported oligonucleotides from the array. The post-synthesis processing reactions, for example, cleaving, purifying and quality control, can be performed after pooling of different supported oligonucleotides. The supports can be removed, if at all, during or after the post-synthesis processing of the pooled different oligonucleotide supports.

The present teachings can be used in pooled assays and can implement pooling of oligonucleotides subsequent to synthesis. The oligonucleotides can be placed in storage, long-term or short-term. The supported oligonucleotides can be stored for longer durations when compared to oligonucleotides that have been subjected to post-processing. Unbound, unsupported, or separated oligonucleotides can be more fragile than supported oligonucleotides. The supported oligonucleotides can be advantageously used immediately or nearly immediately after synthesis, for example, to implement a just-in-time manufacturing process for assay manufacturing. The post-synthesis processes of cleaving, purifying, quality control, and the like, can be performed on multiple oligonucleotides, reducing costs and complexity, accordingly. This manufacturing approach can be utilized for products where the oligonucleotides are pooled post-synthesis, for example, in amplification assays, in polymerase chain reaction assays, in oligonucleotide ligation assays, in SNPLEX (TM) assays, in TAQMAN (TM) assays, or in Universal Probe assays, all of which are available from Applied Biosystems of Foster City, CA.

According to various embodiments, a system is provided including: a plurality of retainment regions, each retainment region being capable of and/or adapted to retain a respective type of an oligonucleotide supported on a respective support; a mixture retainment region; a handling device; a control unit adapted to control the handling device; and a separating unit adapted to simultaneously separate different supported oligonucleotides from respective supports. The system controls the control unit to pool together, in the mixture retainment region, different supported oligonucleotides from different ones of the retainment regions, to form a pool.

According to various embodiments, the system can provide different supported oligonucleotides stored in different ones of the retainment regions. The different supported oligonucleotides can provide a plurality of different types of supported oligonucleotides. The different types of supported oligonucleotides can be respectively disposed in different retainment regions. Each of the different types of supported oligonucleotides can be disposed in two or more different retainment regions. According to various embodiments, the supports including the oligonucleotides can be stacked one over the other or otherwise lined-up in a capillary for retrieval by a handling system.

According to various embodiments, the plurality of retainment regions can be arranged in an addressable array, wherein the control unit can control the handling device to access each retainment region of the addressable array. Access to each retainment region can be provided by movement of the handling device and/or the retainment regions. The control unit can utilize a plurality of addresses corresponding to two or more retainment regions of the addressable array, and can control the transfer of one or more supported oligonucleotide from two or more of the plurality of retainment regions, to the mixture retainment region. The plurality of addresses can be provided using a fixed list, a file, a network transmission, a signal, or any of the methods and devices known in the art. The addresses can be identified by coordinates, a name, or the like.

According to various embodiments, the plurality of retainment regions can comprise a plurality of individually removable containers. Each of the plurality of retainment regions can comprise a machine readable identifier. Alternatively or additionally, at least two of the plurality of retainment regions can comprise a machine readable identifier. The machine readable identifier can identify contents of a single retainment region or a plurality of retainment regions using further mapping methods and devices known in the art.

The system can comprise a mixture of components disposed in a mixture retainment region. The mixture can be delivered before, after, or during the pooling and/or post-synthesis processing of pooled oligonucleotides. The mixture of components can comprise a mixture of supported oligonucleotides. The mixture of components can comprise a mixture of separated, cleaved, or unsupported oligonucleotides. The system can comprise at least one additional mixture retainment region. The mixture retainment region can be a cuvette. The mixture retainment region car, be a vessel disposed in a housing, for example, a tray configured as a micro-titer tray. The housing can have a plurality of retainment regions, wells, chambers, vessels, or the like, therein, for example, 12, 24, 48, 96, 192, 384, or 384 or more, vessels.

According to various embodiments, the handling device can individually handle a single particle having an average diameter of, for example, from about one nanometer to about one centimeter, or from about 10 microns to about 2000 microns, or from about 50 microns to about 1000 microns, or from about 100 microns to about 200 microns. Each support can have an average support diameter of about 1.0 mm or less. The handling unit can comprise a robot, for example, a storage and retrieval robot. The robot can comprise a positioning robot known to those skilled in the art. The teachings of International Publications Nos.: WO 00/49382, international filing date February 15, 2000; WO 00/48735, international filing date February 15, 2000; and WO 03/022437 A1, international filing date September 9, 2002, are all incorporated herein in their entireties by reference. The handling unit can comprise a robot, for example, as described in Noda et al., "Automated Bead Alignment Apparatus Using a Single Bead Capturing Technique for Fabrication of a Miniaturized Bead-Based DNA Probe Array," Analytical Chemistry, Vol. 75, No. 13, July 1, 2003, U.S. Patent Application No. 09/506,870, filed February 15, 2000, or U.S. Patent Application No. 10/211,131, filed August 2, 2002, all of which are incorporated herein in their entireties by reference. According to various embodiments, the handling system can comprise a manual pooling or assembly of different supported oligonucleotides.

According to various embodiments, the system can comprise a detection unit, for example, comprising a field of view that can comprise the mixture retainment region. The system can verify the pooling of different oligonucleotides using a detection image of the mixture region. The image can comprise an image of a supported oligonucleotide as the supported oligonucleotide is being transported by the handling system using a support capture device. An image of the support capture device can be used to verify transfer of the supported oligonucleotide post-delivery. An image of a mixture retainment region can be used to verify transfer of the supported oligonucleotide post-delivery. A plurality of images can be produced by the detection unit.

According to various embodiments, the system can comprise a support removal unit that can remove supports from the mixture retainment region. The system can comprise a storage unit for storing a pool of different separated, unsupported, or cleaved oligonucleotides, and a pool machine-readable identifier. The system can comprise an oligonucleotide synthesizer to synthesize an oligonucleotide, for example, attached to a support.

According to various embodiments, the separating unit can comprise a reagent for chemically cleaving a plurality of different supported oligonucleotides from their respective supports. The cleaving can comprise contacting or washing the supported oligonucleotides with an ammonia bath. Exemplary cleaving components can comprise liquids, solutions, mixtures, or the like. The cleaving components can comprise ammonium hydroxide, ammonia in methanol, mixture of ammonium hydroxide and methylamine, or other chemicals known in the art.

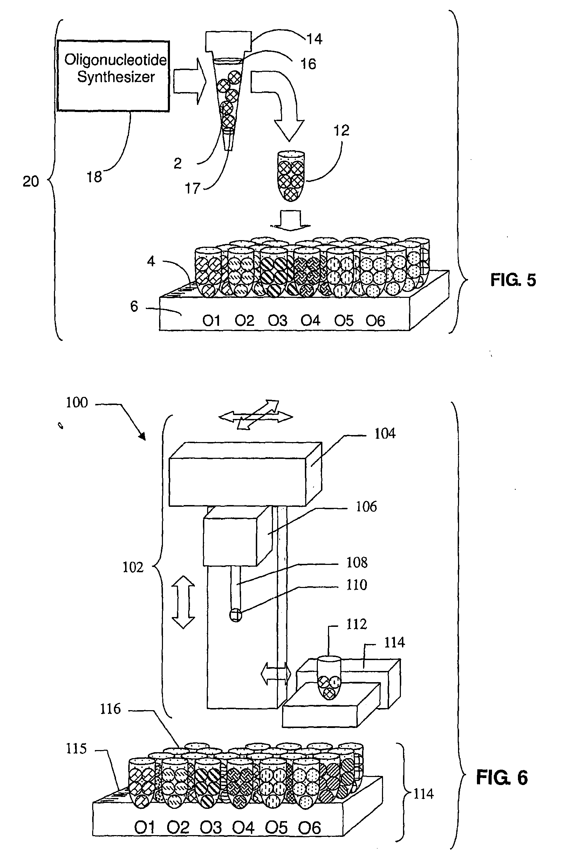

Fig. 5 depicts an arrangement of different supported oligonucleotides retained in a storage system 20. According to various embodiments, an oligonucleotide synthesizer 18 can synthesize oligonucleotides on a support 2, for example, on a bead that can be trapped in a containment region or vessel 14. The vessel 14 can be, for example, a plastic tube, a column, a cuvette, a test tube, a well, a retainment region, a container, or a fluid vessel, for example, those known in the art. The vessel 14 can trap the support 2 using frits 16, 17. The frits 16, 17 can be made using materials suitable as permeable barriers as known in the art, for example, plastic. The vessel 14 can hold a plurality of supports. The plurality of supports can have a volume of, for example, from about one milliliter to about 100 milliliters, from about 50 microliters to about 10 milliliters, from about 100 microliters to about one milliliter, from about 200 microliters to about one milliliter, or from about 200 microliters to about 800 microliters. Each support 2 can have an average particle size diameter, for example, of from about 0.05 mm to about 1 mm, from about 0.1 mm to about 0.5 mm, from about 0.3 mm to about 0.5 mm, or of about 0.4 mm. The vessel 14 can comprise a plurality of supports, for example, about 1,000 supports, about 10,000 supports, about 100,000 supports, about 1,000,000 supports, or more. Each support 2 can be removed from the vessel 14 and disposed in a retainment region 12, for example, a tube having a diameter of about 3 millimeters and a length of about 10 millimeters, a well, a vial, or any other fluid vessel. The retainment region 12 can comprise at least one retainment support and/or at least one frame. The retainment region 12 can be disposed in an array of retainment regions 6. The retainment region 12 and/or the array 6 can comprise a machine readable identifier 4. The vessel 14 can be used as a retainment region 12. The array of retainment regions 6 can be grouped into a housing, for example, into a microtiter tray or other device having the footprint of a microtiter tray. The array of retainment regions 6 can be positioned adjacent other arrays of retainment regions (not shown). Each of the other arrays of retainment regions can comprise a machine readable identifier. The array 6 can comprise a plurality of vessels 14. The array 6 can be stored before and/or after use.

Fig. 6 is a schematic diagram of a handling system according to various embodiments. The handling system can comprise a storage and retrieval robot (SRR) 100 and can use linear motors 104, 106, 114 to move a robot head 102 in three dimensions, as depicted by the arrows in Fig. 6. The robot head 102 can be positioned over any or all retainment regions 116. The retainment regions 116 can be comprised in an addressable array 114. The robot head 102 can use any or all of the linear motors 104, 106, 114 in various combinations to locate a support capture device 108 into or above contents of retainment region 116, and subsequently move a supported oligonucleotide 110 into a mixture retainment region 112 using the support capture device 108. The robot head can select oligonucleotides from a vessel, for example, from vessel 14 shown in Fig. 5.

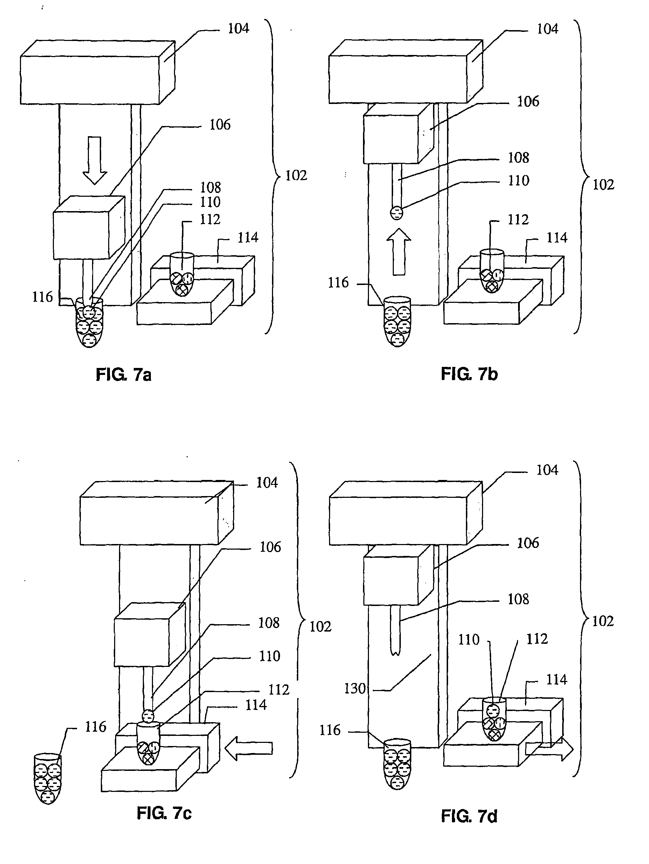

According to various embodiments, Fig. 7a, Fig. 7b, Fig. 7c, and Fig. 7d illustrate four steps that can be used to retrieve the support 110 from the retainment region 116 and deliver the support 110 to the mixture retainment region 112. In Fig. 7a, one or more of the linear motors 104, 106, 114 can move the support capture device 108 in alignment with a retainment region 116. According to various embodiments, subsequent to positioning and aligning the support capture device 108 in line with the retainment region 116 a distal end of the support capture device 108 can be immersed in the retainment region 116, placing the distal tip in contact with a buffer (not shown) and a plurality of supports. The distal end can be positioned at the bottom of the retainment region 116. The distal end of the retainment region 116 can be held at the bottom of the support-stocked retainment region 116, for a desired time period, for example, at least about 0.1 second, at least about 0.5 second, at least 1 second, at least about 2 seconds, or at least about 10 seconds. A support attraction force, for example, a magnetic force, a suction force, or a vacuum, can be applied to the support capture device 108. The force can be used to remove a desired number of supports from the retainment region 116, and/or from the support capture device 108. The number of supports retained by the support capture device 108 can be varied, for example, 1, 2, or 4 supports can be captured at a time. The support capture device 108 can have an opening at the distal end thereof, and the opening can have an inner diameter that is smaller than the average particle size diameter of the supports. The support capture device 108 can comprise a protective sleeve (not shown) to protect a support or supports during transport from the retainment region 116 to the mixture retainment region 112. The contents of the retainment region 116 can be identified using a machine readable identifier 115 (shown in Fig. 6). The retainment region 116 in Figs. 7a, 7b, 7c, and 7d is depicted without the array 114 shown in Fig. 6, for the sake of simplicity.

As shown in Fig. 7b one or more of the linear motors 104, 106, 114 can direct the support capture device 108, while holding a captured support 110, away from a respective retainment region 116.

As shown in Fig. 7c the robot head 102 can be positioned over the mixture retainment region 112 such that the mixture retainment region 112 is aligned with the support capture device 108. The mixture retainment region can receive the support 110 upon application of a support releasing force to the support capture device 108. The support release force can be a puff of air, for example, or the interruption of a vacuum or suction. The support release force can be gravity subsequent to disabling a support-attracting force. The alignment of the robot head 102, the support capture device 108, and the mixture retainment region 112, can be accomplished by moving the robot head 102, moving the mixture retainment region 112, and/or moving both the robot head 102 and the mixture retainment region 112.

Fig. 7d illustrates a position of the robot head 102 and the mixture retainment region 112 after the support 110 has been delivered to the mixture retainment region 112. As shown, the distal tip 130 of the support capture head 102 can comprise a concave or recessed shape.

According to various embodiments, the handling system can repeat the process illustrated by Fig. 7a, Fig. 7b, Fig. 7c, and Fig. 7c until different oligonucleotides, desired to assemble an assay, have been retrieved from the plurality of respective retainment regions 116 in array 114, and pooled into the mixture retainment region 112.

According to various embodiments, the handling system can be controlled using software implemented on a computer system that interfaces with and controls the handling system. The software can map an oligonucleotide identifier to an addressable location of the array 114. The mapping can comprise, for example, translating a colloquial oligonucleotide name to an addressable location, relating a colloquial assay name to various different oligonucleotides comprising the assay, and/or translating a storage device address location, where the storage device stores the supported oligonucleotides upon synthesis by an oligonucleotide synthesizer into addresses for the handling system. According to various embodiments, the oligonucleotide synthesizer can use the storage device to place the different oligonucleotides in different retainment regions.

According to various embodiments, subsequent requests for a particular assay need not wait for the synthesis and preparation of the supported oligonucleotides needed. Instead, the supported oligonucleotides can be located in respective retainment regions and can be immediately or nearly immediately available. Subsequent copies of an assay can be extremely low cost or essentially free. The cost of post-synthesis processing of the stored supported oligonucleotides can be reduced by the multiplex process of the present teachings.

The system of the present teachings can connect to an inventory control system. As different stored supported oligonucleotides are consumed, the inventory system can update necessary counts. The inventory control system can request particular oligonucleotides, for example, by sending instructions to the oligonucleotide synthesizer, or by notifying an operator. Various thresholds known in the art can cause requests for more oligonucleotides to be generated, for example, if inventory of a particular oligonucleotide falls below a certain percentage relative to the storage capacity of the system. The threshold can be, for example, about 20%, about 10%, about 5%, or about 1%. The inventory control system can track an oligonucleotide freshness date. The inventory control system can order expunging of oligonucleotides upon freshness expiration or for one or more other reasons. The inventory control system can order supplies for the oligonucleotide synthesizer as supported oligonucleotides are expunged.

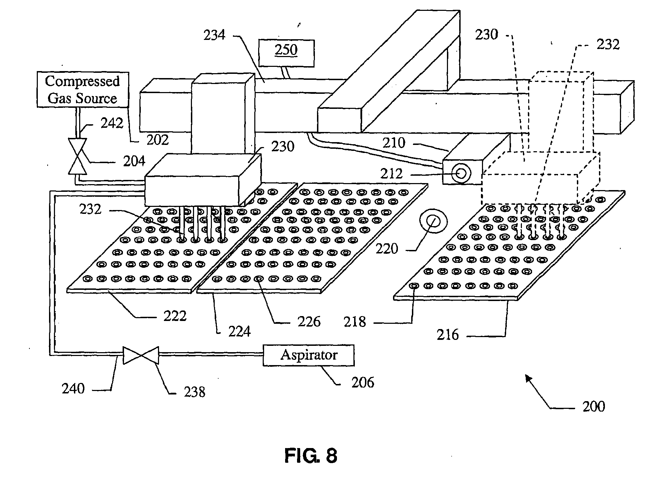

Fig. 8 illustrates a storage and retrieval system 200 according to various embodiments. The storage and retrieval system 200 can comprise a plurality of storage arrays 222, 224 including retainment regions 226. The storage arrays 222, 224 can be, for example, two 384-well microtiter plates storing 768 sets of supported oligonucleotides, where the supported oligonucleotides can be identified by respective positions of the retainment regions 226. The robot head 230 can use a support capture device 232, also known as a micro vacuum tweezer, capillary tube, or suction tube. The robot head 230 can travel along a rail 234 to deliver supports retrieved from storage arrays 222, 224 and can deliver them into a mixture retainment region 218 in a mixture retainment region array 216. The robot head 230 can comprise a plurality of support capture devices 232. For example, the robot head 230 can hold 16 support capture devices 232 with an inner diameter (i.d.) of 50 µm, available from GL Science, Japan, where the capillaries can be mounted onto the robot head 230 through inner seal connectors (not shown), available from GL Science, Japan. The support capture devices 232 can be aligned in the pitch of the retainment regions 226 of a 384-well microtiter plate, a retainment region pitch of about 4.5 mm.

The robot head 230 can traverse a field of view of a detector system 210 that can comprise a radiation transmitter 212. Radiation emitted from the radiation transmitter 212 can impinge upon one or more supports, while stationed or while being transferred by the respective support capture devices 232. The supports can travel across the field of view. A control system 250 can interpret results detected by the detector and determine whether a support has been retrieved by the support capture device 232. According to various embodiments, the detection system can comprise a detector (not shown), adjacent or sharing a same housing as the radiation transmitter 212, can be embedded in the radiation source 212, and the detector can be located in the detection system 210. Alternatively, or additionally, a detector can be placed along an emitted radiation path that passes through a detection region 220. For example, a detector system, vision sensor model CV-700 available from Keyence Corporation of Woodcliff Lake, NJ, can be used to detect whether a single support is captured by one or each of the support capture devices 232. A ringshaped light from white light emitting diodes (not shown), model CA-DRW3, available from Keyence Corporation of Woodcliff Lake, NJ, and can be used to illuminate the support capture devices 232. A desired brightness threshold value for the support image, supports and background, including the support capture devices 232 can be established such that the existence of single supports on the support capture devices 232 can be detectable.

The storage and retrieval system 200 can comprise a compressed gas source 202 and an aspirator 206 that can create a vacuum or suction. The aspirator 206 can be capable of creating suction at the distal ends of the support capture devices 232. The gas line from the robot head 230 to the aspirator 206 can comprise a valve 238. The gas line 242 from the compressed gas source to the aspirator 206 can comprise a valve 204. The aspirator 206 can comprise a liquid source such as running water. The valves 204, 238 can be computer controlled. The gas lines 240, 242 can be subdivided, as necessary, to deliver suction or compressed gas to one or more of the plurality of support capture devices 232. The valves 204, 238 can be added in multiples as necessary to control subdivisions of gas lines 240, 242. For example, the aspirator 206 can be a model A-3S aspirator, available from Tokyo Rikakikai of Japan, and the aspirator 206 can evacuate the inside of the support capture devices 232. The compressed gas lines 242 can produce high pressure in the inside of the support capture devices 232, and the high pressure can be used to release the support. The capturing and releasing of supports on the support capture devices 232 can be controlled by alternatively communicating the vacuum or compressed gas to the support capture devices 232. A buffer, for example, water, can be supplied to the support capture devices 232 by using a syringe pump (not shown), for example, model sp-230iw available from WPI, U.S.A.

A method is provided, comprising: pooling together a plurality of supported oligonucleotides to form a mixture, and simultaneously separating the oligonucleotides of the supported oligonucleotides in the mixture from their supports to form a pool of separated oligonucleotides. Each supported oligonucleotide can comprise an oligonucleotide attached to a support The method further comprise packaging the pool of separated oligonucleotides in a container. The container can be a tube, vial, or the like. The packaging can comprise providing information related to the pool of separated oligonucleotides. The plurality of supported oligonucleotides can comprise a plurality of different supported oligonucleotides. The pooling can comprise manipulating a robot to handle respective supports for the plurality of the supported oligonucleotides while supported oligonucleotides are supported on their respective supports. The pooling can comprise transferring the plurality of supported oligonucleotides supported on their supports to the mixture from a plurality of different respective retainment regions. The separating can comprise chemically cleaving a plurality of different oligonucleotides from their respective support. The pool of separated oligonucleotides can comprise a homogeneous reaction mixture. The homogeneous reaction mixture can comprise components for conducting a fluorogenic 5' nuclease assay.

According to various embodiments, a method for facilitating genetic analysis is provided comprising: receiving from a user a request for one or more genetic analysis assays; formulating each assay; and providing to the user (i) the one or more assays, with each assay being provided in a single tube format, and (ii) information related to the one or more assays, with the information being in electronic form. The formulating can comprise pooling together into a mixture retainment region a plurality of different supported oligonucleotides each supported on a respective support, and simultaneously separating or cleaving the different oligonucleotides from their respective supports to form a mixture of separated oligonucleotides. The separating or cleaving can occur, for example, in the mixture retainment region or at a different location.

According to various embodiments, the method can comprise packaging together the different separated oligonucleotides. The different separated oligonucleotides can be packaged together in the mixture retainment region. The different separated oligonucleotides can be packaged together in a vessel that differs from the mixture retainment region. The different supports for the respective plurality of different separated oligonucleotides can be packaged together with the different separated oligonucleotides.

According to various embodiments, the method can provide shipping the packaged different separated oligonucleotides to a user. The package can comprise a data storage medium that contains data about the contents of the package. At least one of the one or more assays and the information can be shipped to the user together in a single package. The information can be comprised on a disk. The information can be provided by a computer network. The information can be transmitted to the user via electronic mail. The information can comprise, at least in part, data formatted using American Standard Code for Information Exchange (ASCII).

According to various embodiments, the pooling of different oligonucleotides can comprise manipulating a robot to handle respective supports for the plurality of the different oligonucleotides, while the different oligonucleotides are supported by or attached to their respective supports. The pooling can comprise transferring the plurality of different supported oligonucleotides to the mixture retainment region from a plurality of different respective retainment regions.

According to various embodiments, the method can comprise formulating one or more assays, where each assay can comprise a homogeneous reaction mixture. The homogeneous reaction mixture can comprise components for conducting a fluorogenic 5' nuclease assay or a fluorogenic 3' nuclease assay.

According to various embodiments, the method can comprise assembling or formulating an oligonucleotide ligation assay. The method can comprise assembling or formulating at least three different or unique oligonucleotides for each assay. The assembling or formulating can comprise selecting and transferring a locus specific oligonucleotide comprising a single probe. The assay can be allele specific. The assay can be a heterozygote assay. The assay can be a single allele specific oligonucleotide or a plurality of alleles including specific oligonucleotides. The assay can comprise fluorescent markers, for example, dyes or nanoparticles. The assay can be subjected to PCR or any other type of nucleotide amplification process known in the art.

According to various embodiments, the present teachings can provide one or more of the following benefits: automating the storage and retrieval of oligonucleotides; reduction of synthesis cost to near zero after the first customer, reduction of post-synthesis costs by multiplexing different oligonucleotides in a mixture retainment region; supported oligonucleotides are less susceptible to losses by evaporation or splattering; supported oligonucleotides are less susceptible to changing concentration; for each kind of support users can order an assay that comprises any number of supports, for example, from about one to about 1000 of the same type of oligonucleotide support, and thus users can specify the concentration of an oligonucleotide in the assay; reduction of cost in SNP oligonucleotide orders; and easier implementation of an assembly line, for example, by using one or more compact robot implementing proven support technology.

Other embodiments of the present teachings will be apparent to those skilled in the art from consideration of the present specification and practice of the teachings disclosed herein. It is intended that the present specification and examples be considered as exemplary only.

## Claims

1. A system comprising:
a plurality of retainment regions, each retainment region being adapted to retain a respective type of oligonucleotide supported on a respective support;
a mixture retainment region;
a handling device;
a control unit programmed to control the handling device to pool in the mixture retainment region different supported oligonucleotides from different ones of the retainment regions, to form a pool; and
a separating unit adapted to simultaneously separate different types of oligonucleotides supported on different respective supports, from their respective supports.

2. The system of claim 1, further comprising different supported oligonucleotides in different ones of the retainment regions.

3. The system of claim 1, wherein the different supported oligonucleotides comprise a plurality of different types of supported oligonucleotides.

4. The system of claim 3, wherein the different types of supported oligonucleotides are respectively disposed in different retainment regions.

5. The system of claim 3, wherein each of the different types of supported oligonucleotides is disposed in two or more different retainment regions.

6. The system of claim 1, wherein the plurality of retainment regions are arranged in an addressable array and the control unit is programmed to control the handling device to access a plurality of retainment regions of the addressable array.

7. The system of claim 6, wherein access to each retainment region is provided by movement of the handling device.

8. The system of claim 6, wherein the control unit is adapted to receive a plurality of addresses corresponding to two or more retainment regions of the addressable array, and transfer one or more supported oligonucleotides from two or more of the plurality of retainment regions, to the mixture retainment region.

9. The system of claim 1, further comprising a purification unit that is adapted to purify the pool.

10. The system of claim 9, further comprising a mixture of components disposed in the mixture retainment region.

11. The system of claim 10, wherein the mixture of components comprises a mixture of supported oligonucleotides.

12. The system of claim 10, wherein the mixture of components comprises a mixture of separated oligonucleotides.

13. The system of claim 1, wherein the handling device is adapted to individually handle a single particle having a diameter of from about 10 microns to about 2000 microns.

14. The system of claim 1, wherein the handling device is adapted to individually handle a single particle having a diameter of from about 50 microns to about 1000 microns.

15. The system of claim 1, wherein the handling device is adapted to individually handle a single particle having a diameter of from about 100 microns to about 200 microns.

16. The system of claim 1, wherein each support has an average support diameter of about 1.0 mm or less.

17. The system of claim 1, further comprising a detection unit that comprises a field of view that includes the mixture retainment region.

18. The system of claim 17, wherein the system is adapted to verify the pooling of different oligonucleotides using a detection image of the mixture region, produced by the detection unit.

19. The system of claim 1, wherein the plurality of retainment regions comprises a plurality of individually removable containers.

20. The system of claim 1, wherein each of the plurality of retainment regions comprises a machine readable identifier.

21. The system of claim 1, further comprising at least one additional mixture retainment region.

22. The system of claim 1, wherein at least two of the plurality of retainment regions comprises a machine readable identifier.

23. The system of claim 1, further comprising a support removal unit adapted to remove supports from the mixture retainment region.

24. The system of claim 1, wherein the mixture retainment region is a cuvette.

25. The system of claim 1, wherein the mixture retainment region is a vessel disposed in a housing, wherein the housing comprises the footprint of a microtiter tray.

26. The system of claim 1, wherein the separating unit comprises a reagent for chemically cleaving a plurality of different oligonucleotides from their respective supports.

27. The system of claim 1, wherein said cleaving comprises reacting the plurality of different oligonucleotides with at least one of ammonium hydroxide, ammonia in methanol, a mixture of ammonium hydroxide and methylamine, or a combination thereof.

28. The system of claim 1, further comprising a storage unit for storing a pool of different separated oligonucleotides, and a pool machine-readable identifier.

29. The system of claim 1, further comprising an oligonucleotide synthesizer capable of synthesizing an oligonucleotide attached to a support.

30. The system of claim 1, wherein the handling unit comprises a robot.

31. A system comprising:
a plurality of retainment regions, each retainment region being adapted to retain a respective type of chemical chemically bonded to a respective support;
a mixture retainment region;
a handling device;
a control unit programmed to control the handling device to pool in the mixture retainment region different supported chemicals from different ones of the retainment regions, to form a pool; and
a separating unit adapted to simultaneously separate different types of chemicals chemically bonded to different respective supports, from their respective supports.

32. The system of claim 31, further comprising different types of chemicals chemically bonded to respective supports, in different ones of the retainment regions.

33. The system of claim 31, wherein the plurality of retainment regions are arranged in an addressable array and the control unit is programmed to control the handling device to access a plurality of retainment regions of the addressable array.

34. The system of claim 33, wherein the control unit is adapted to receive a plurality of addresses corresponding to two or more retainment regions of the addressable array, and transfer one or more supported chemicals from two or more of the plurality of retainment regions, to the mixture retainment region.

35. A method comprising:
pooling together a plurality of supported oligonucleotides to form a mixture, each supported oligonucleotide comprising an oligonucleotide attached to a support; and
simultaneously separating the oligonucleotides of the supported oligonucleotides in the mixture from their supports to form a pool of separated oligonucleotides.

36. The method of claim 35, further comprising packaging the pool of separated oligonucleotides in a container.

37. The method of claim 36, wherein the container is a tube or vial.

38. The method of claim 36, wherein said packaging further comprises providing information related to the pool of separated oligonucleotides.

39. The method of claim 35, wherein the plurality of supported oligonucleotides comprises a plurality of different supported oligonucleotides.

40. The method of claim 35, wherein said pooling further comprises manipulating a robot to handle respective supports for the plurality of the supported oligonucleotides while supported oligonucleotides are supported on their respective supports.

41. The method of claim 35, wherein said pooling comprises transferring the plurality of supported oligonucleotides supported on their supports to the mixture from a plurality of different respective retainment regions.

42. The method of claim 35, wherein said separating comprises chemically cleaving a plurality of different oligonucleotides from their respective support.

43. The method of claim 35, wherein the pool of separated oligonucleotides comprises a homogeneous reaction mixture.

44. The method of claim 35, wherein the homogeneous reaction mixture comprises components for conducting a fluorogenic 5' nuclease assay.

45. A method for facilitating genetic analysis, comprising:
receiving from a user a request for one or more genetic analysis assays;
formulating each assay, said formulating comprising
pooling together into a mixture retainment region a plurality of different oligonucleotides each supported on a respective support, and
simultaneously separating the different oligonucleotides from their respective supports in the mixture retainment region; and
providing to the user the one or more assays.

46. The method of claim 45 further comprising, packaging the different separated oligonucleotides together.

47. The method of claim 46, wherein the different separated oligonucleotides are packaged together in the mixture retainment region.

48. The method of claim 46, wherein the different separated oligonucleotides are packaged together in a vessel and the vessel is different than the mixture retainment region.

49. The method of claim 45, wherein different respective supports for the plurality of different oligonucleotides are packaged with the different separated oligonucleotides.

50. The method of claim 45, wherein said separating comprises chemically cleaving a plurality of different oligonucleotides from their respective support.

51. The method of claim 50, wherein providing to the user comprises providing each assay in a single tube format, and providing to the user information related to the one or more assays.

52. The method of claim 45, wherein said pooling further comprises manipulating a robot to handle respective supports for the plurality of the different oligonucleotides while different oligonucleotides are supported on their respective supports.

53. The method of claim 45, wherein said pooling comprises transferring the plurality of different oligonucleotides supported on their supports to the mixture retainment region from a plurality of different respective retainment regions.

54. The method of claim 46, further comprising shipping the packaged different separated oligonucleotides to the user.

55. The method of claim 46, wherein the packaging is packaged with a data storage medium including data about the contents of the package vessel.

56. The method of claim 45, wherein the one or more assays each comprises a homogeneous reaction mixture.

57. The method of claim 56, wherein the homogeneous reaction mixture comprises components for conducting a fluorogenic 5' nuclease assay.

58. The method of claim 45, wherein the information is provided on a disk.

59. The method of claim 45, wherein the information is provided by a computer network.

60. The method of claim 45, wherein the information is transmitted to the user via electronic mail.

61. The method of claim 45, wherein the information is comprised, at least in part of American Standard Code for Information Exchange (ASCII) formatted text.

62. The method of claim 45, wherein at least one of the one or more assays and the information are shipped to the user together in a single package.

63. A method comprising:
pooling together into a mixture retainment region a plurality of different types of chemicals, each type of chemical being chemically bonded to a respective support, and simultaneously cleaving the different types of chemicals from their respective supports in the mixture retainment region, to form a pool of cleaved chemicals.

64. The method of claim 63, further comprising packaging together the different types of cleaved chemicals.
